# EUROPEAN PATENT APPLICATION

(11) **EP 4 484 962 A1**
(43) Date of publication of application: **01.01.2025**
(21) Application number: 23779148.8
(22) Date of filing: 28.02.2023
(51) Int. Cl.: G01N 35/00, G01N 35/02, G01N 35/10, C12Q 1/6851, G01N 33/53, C12M 1/00

(54) **TEST CONTAINER AND NUCLEIC ACID TEST METHOD**

(30) Priority: 30.03.2022 JP 2022057525
(71) Applicant: FUJIFILM Corporation, Tokyo 106-8620 (JP)
(72) Inventor: HAMA, Takeshi, Ashigarakami-gun Kanagawa 258-8538 (JP); ETO, Daisuke, Ashigarakami-gun Kanagawa 258-8538 (JP); ISHII, Hiroyasu, Ashigarakami-gun Kanagawa 258-8538 (JP); ISOZAKI, Naoto, Ashigarakami-gun Kanagawa 258-8538 (JP); KOMIYAMA, Kazuoki, Ashigarakami-gun Kanagawa 258-8538 (JP)
(74) Representative: Dehns Germany Partnerschaft mbB
(86) International application number: PCT/JP2023/007397
(87) International publication number: WO 2023/189118

(57) **Abstract**

A test container, which is attached to a centrifuge and in which a sample solution is fed from a first chamber to a second chamber in order by a centrifugal force caused by a rotation of the centrifuge, includes: a liquid reservoir portion that stores the sample solution put in from an inlet in front of the first chamber; a valve that includes a liquid storage portion temporarily storing the sample solution to be fed from the first chamber to the second chamber and is capable of switching between a first state where the first chamber and the liquid storage portion communicate with each other and the second chamber and the liquid storage portion do not communicate with each other, a second state where the second chamber and the liquid storage portion communicate with each other and the first chamber and the liquid storage portion do not communicate with each other, and a third state where both of the first chamber and the second chamber do not communicate with the liquid storage portion; and a sealing part that prevents a back flow of the sample solution after the sample solution is fed from the liquid reservoir portion to the first chamber.

## Description

### BACKGROUND OF THE INVENTION

### 1. Field of the Invention

The present invention relates to a test container and a nucleic acid test method.

### 2. Description of the Related Art

A test container, such as a test cartridge or an analysis chip, which is used to perform various analyses on a sample extracted from a biological specimen, is known. For example, the test container includes a plurality of chambers (liquid storage portions) that store liquid and flow channels that connect the chambers. In the test container, a sample solution is fed from the chamber to the chamber using an external force, such as electromagnetic force, a centrifugal force, or pressure.

JP2019-515308A discloses a microfluidic chip that feeds a specimen (a sample solution) put in from a specimen inlet (an inlet) provided at one end of a specimen chamber from the specimen chamber to a mixing chamber with a centrifugal force. The microfluidic chip disclosed in JP2019-515308A includes a circuit, that is, a vent flow channel, which is adapted to provide an air connection between the specimen chamber and the mixing chamber, to be capable of smoothly feeding liquid.

### SUMMARY OF THE INVENTION

In the microfluidic chip disclosed in JP2019-515308A, fluid is fed from the specimen chamber provided on an upstream side to the mixing chamber provided on a downstream side and the flow channel between both the chambers then remains open. In a case of an analysis that requires treatment at a heating temperature of 90°C or higher in the downstream chamber, the sample solution may be vaporized and flow back to the upstream chamber. For this reason, a test error caused by a shortage in the amount of the sample solution in the downstream chamber, or the like may occur. JP2019-515308A discloses that heating treatment is performed in the downstream chamber to which the sample solution is fed for pretreatment or the like of the sample solution. However, since a heating temperature is as low as about 65°C, there is no consideration for a problem of the back flow of the sample solution to the upstream chamber which is caused by the vaporization of the sample solution.

The present disclosure has been made in view of the above circumstances, and an object of the present disclosure is to provide a test container and a nucleic acid test method that can suppress the back flow of a sample solution from a downstream chamber to an upstream chamber in a test container for feeding liquid with a centrifugal force.

A test container according to the present disclosure is a test container including an inlet into which a sample solution is to be put, a first chamber in which the sample solution to be fed from the inlet is stored, and a second chamber in which the sample solution to be fed from the first chamber is stored. The test container is attached to a centrifuge and the sample solution is fed from the first chamber to the second chamber in order by a centrifugal force caused by a rotation of the centrifuge. The test container comprises: an attachable and detachable lid part that covers the inlet; a liquid reservoir portion that stores the sample solution put in from the inlet in front of the first chamber; a valve that is disposed between the first chamber and the second chamber, includes a liquid storage portion temporarily storing the sample solution to be fed from the first chamber to the second chamber, and is capable of switching between a first state where the first chamber and the liquid storage portion communicate with each other and the second chamber and the liquid storage portion do not communicate with each other, a second state where the second chamber and the liquid storage portion communicate with each other and the first chamber and the liquid storage portion do not communicate with each other, and a third state where both of the first chamber and the second chamber do not communicate with the liquid storage portion; and a sealing part that prevents a back flow of the sample solution after the sample solution is fed from the liquid reservoir portion to the first chamber.

It is preferable that the sealing part is a hydrophobic substance having a specific gravity lower than that of the sample solution.

It is preferable that the hydrophobic substance is a low-polarity solvent or a thermoplastic substance.

It is preferable that the hydrophobic substance is provided in the liquid reservoir portion or the first chamber.

The test container may further comprise a hydrophobic substance storage portion that is provided on a side closer to a rotation axis of the centrifuge than the liquid reservoir portion and stores the hydrophobic substance.

It is preferable that, in a case where a volume of the liquid reservoir portion is denoted by V1, a volume of the first chamber is denoted by V2, a volume of the liquid storage portion is denoted by V3, and a volume of the second chamber is denoted by V4, a relationship of "V1 ≥ V2 ≥ V3 ≥ V4" is satisfied.

A reagent to react with the sample solution may be stored in the first chamber, the liquid storage portion, or the second chamber.

A reagent that contains an amplification reagent for amplifying a specific nucleic acid sequence and a probe for determining a nucleic acid sequence can be used as the reagent.

The probe may be a fluorescent probe.

A nucleic acid test method according to the present disclosure is a nucleic acid test method using the test container according to the present disclosure in which the reagent is stored in the liquid storage portion. The test method comprises: putting the sample solution in from the inlet of the test container in an initial state where the valve is in the second state or the third state, to store the sample solution in the liquid reservoir portion; hermetically sealing the inlet with the lid part; feeding the sample solution from the liquid reservoir portion to the first chamber with a centrifugal force; heating the first chamber to perform heating treatment of the sample solution in a state where a back flow of the sample solution from the first chamber to the liquid reservoir portion is prevented by the sealing part; switching the valve to the first state; feeding the sample solution from the first chamber to the liquid storage portion of the valve with a centrifugal force to mix the sample solution and the reagent in the liquid storage portion; switching the valve to the second state; feeding a liquid mixture of the sample solution and the reagent from the liquid storage portion to the second chamber with a centrifugal force; switching the valve to the third state or the first state; controlling a temperature of the liquid mixture in the second chamber to amplify the specific nucleic acid sequence contained in the liquid mixture; irradiating the liquid mixture with excitation light to detect fluorescence generated from the fluorescent probe; and determining whether or not the specific nucleic acid sequence is present or a concentration of the specific nucleic acid sequence.

According to the test container and the nucleic acid test method of the present disclosure, it is possible to suppress the back flow of a sample solution from a downstream chamber to an upstream chamber.

### BRIEF DESCRIPTION OF THE DRAWINGS

Fig. 1 is a perspective view showing a schematic configuration of a test container 10 according to an embodiment.
Fig. 2A is a schematic cross-sectional view of the test container 10, and Fig. 2B is a schematic plan view of a flow channel structure.
Figs. 3A and 3B show a state where the test container 10 is switched to a first state by a valve 20, in which Fig. 3A is a schematic cross-sectional view of the test container 10 and Fig. 3B is a schematic plan view of a flow channel structure.
Figs. 4A and 4B show a state where the test container 10 is switched to a second state by the valve 20, in which Fig. 4A is a schematic cross-sectional view of the test container 10 and Fig. 4B is a schematic plan view of the flow channel structure.
Fig. 5 is a diagram showing an aspect of the test container 10.
Fig. 6 is diagrams showing states of the flow channel structure 11 before and after a sample solution is fed from a liquid reservoir portion to a first chamber.
Fig. 7 is diagrams showing states of a flow channel structure 11A of a modification example before and after the sample solution is fed from a liquid reservoir portion to a first chamber.
Fig. 8 is a schematic view showing the test container provided with a reagent.
Fig. 9 is a schematic plan view of a test container 50 of a modification example.
Fig. 10 is a schematic plan view of a test container 52 of a modification example.
Fig. 11 is diagrams showing states of a flow channel structure 11B of the test container 52 before and after the sample solution is fed from a liquid reservoir portion to a first chamber.
Fig. 12 is a schematic plan view of a test container 53 of a modification example.
Fig. 13 is diagrams showing states of a test container 54 of a modification example before and after the sample solution is fed from a liquid reservoir portion to a first chamber.
Fig. 14 is a diagram showing a schematic configuration of a test device 100 according to an embodiment.
Fig. 15 is a diagram showing a schematic configuration of a test device 101 of a modification example.
Fig. 16 is a diagram showing a schematic configuration of a test device 102 of a modification example.
Fig. 17 is a diagram showing steps of a nucleic acid test (Part 1).
Fig. 18 is a diagram showing the steps of the nucleic acid test (Part 2).
Fig. 19 is a diagram showing steps of a β-glucan test (Part 1).
Fig. 20 is a diagram showing the steps of the β-glucan test (Part 2).

### DESCRIPTION OF THE PREFERRED EMBODIMENTS

Embodiments of the present disclosure will be described in detail below with reference to the drawings. The scale and the like of each component shown in the drawings are appropriately changed from the actual ones. In each drawing, the same components are denoted by the same reference numerals.

### "Test container"

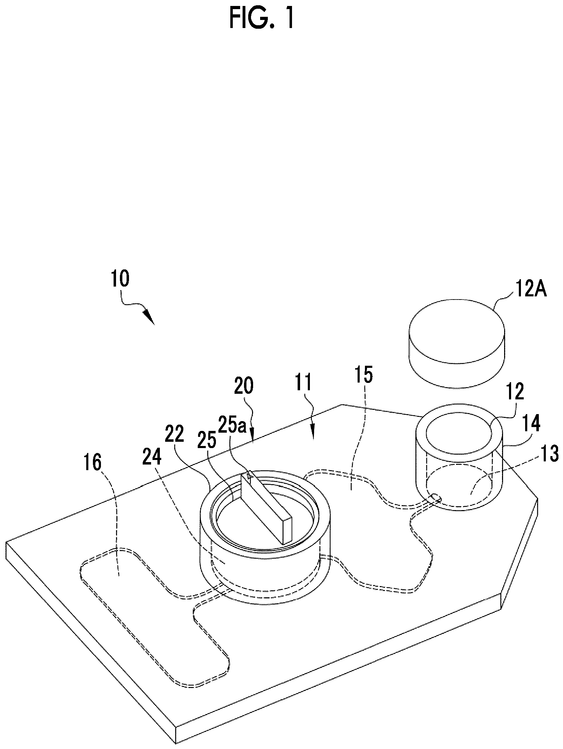

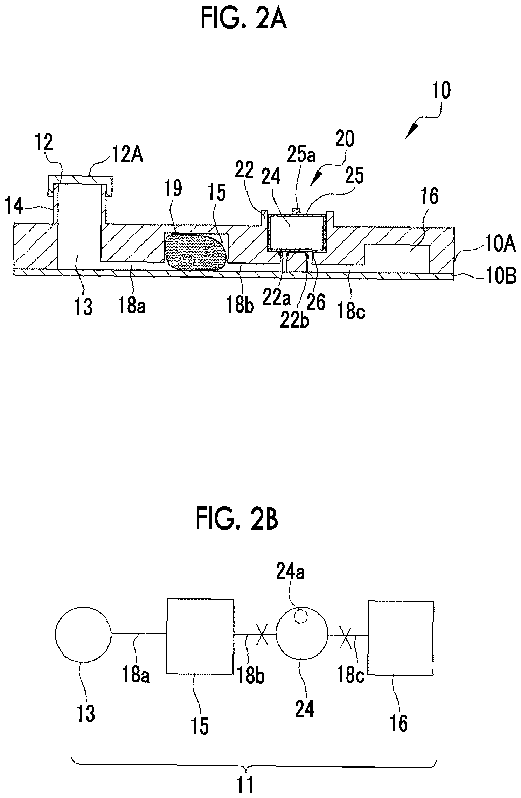

Fig. 1 is a perspective view schematically showing a test container 10 according to an embodiment. Fig. 2A is a schematic cross-sectional view of the test container 10, and Fig. 2B is a schematic plan view showing a flow channel structure 11 of the test container 10 in a simplified manner.

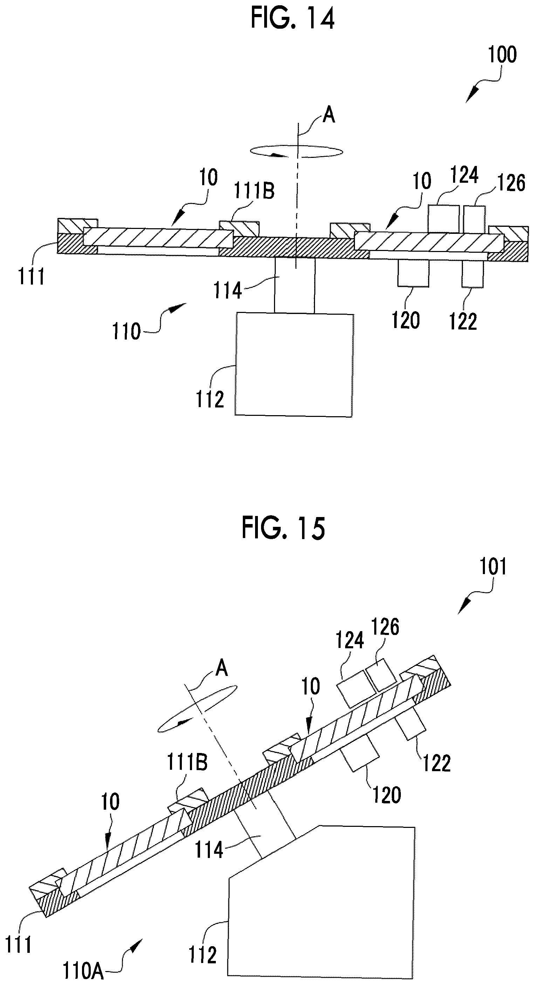

The test container 10 is a microchannel device including a flow channel structure 11 that includes a plurality of chambers and flow channels connecting the chambers. The test container 10 is attached to a centrifuge, and liquid in the test container 10 is transferred from the chamber to the chamber by a centrifugal force caused by the rotation of the centrifuge. The test container 10 of this example is a test container that is loaded into a test device 100 (see Fig. 14) comprising a centrifuge and is used for a biochemical analysis test.

The test container 10 includes a body member 10A in which recessed portions, holes, and the like forming a part of the flow channel structure 11 including the flow channels and the chambers are formed, and a bottom member 10B that forms bottom surfaces of the flow channels (see Figs. 2A and 2B).

As long as a material of the body member 10A is a publicly known resin molding plastic material, the material can be used without being particularly limited. However, polycarbonate, polypropylene, cycloolefin, or a silicone resin is preferable from the viewpoint of heat resistance and transparency.

The bottom member 10B is formed of, for example, a thin plate or a film. As long as a material of the bottom member 10B is a publicly known resin molding plastic material, the material can be used without being particularly limited. However, the same material as the body member 10A is preferable from the viewpoint of adhesiveness to the body member 10A. Although details will be described later, from the viewpoint of being capable of improving adhesion to a heating unit and efficiently performing heating in a case where a first chamber 15 and a second chamber 16 are to be heated, it is preferable that the bottom member 10B is formed of a film. Portions of the body member 10A that form the first chamber 15 and the second chamber 16 close to an upper surface may also be formed of a film.

The test container 10 comprises an inlet 12, a lid part 12A, a liquid reservoir portion 13, a first chamber 15, a second chamber 16, a valve 20 that is disposed between the first chamber 15 and the second chamber 16, flow channels 18a to 18c that connect the liquid reservoir portion 13, the first chamber 15, and the second chamber 16, and a sealing part 19. The plurality of chambers comprised in the test container 10 are the liquid reservoir portion 13, the first chamber 15, the second chamber 16, and a liquid storage portion 24. Hereinafter, in a case where it is not necessary to distinguish the liquid reservoir portion 13, the first chamber 15, the second chamber 16, and the liquid storage portion 24, the liquid reservoir portion 13, the first chamber 15, the second chamber 16, and the liquid storage portion 24 may be collectively referred to as a chamber.

The inlet 12 is an opening into which a sample solution 40 is to be put. The lid part 12A is a lid part that covers the inlet 12 and can be attached to and detached from the opening of the inlet 12. In this example, the lid part 12A is formed to be capable of being screwed to an end portion of a tubular portion 14 that forms the inlet 12. Methods of attaching and detaching the lid part 12A are not particularly limited, and the lid part 12A may be attached to and detached from the tubular portion 14 using, for example, a snap type cap structure or a pressure sensitive adhesive. The lid part 12A allows the inlet 12 to be open in a case where the sample solution is to be put in, but closes the inlet 12 to exclude the mixing of contaminants from the outside and to prevent the vaporization of the sample solution 40 from the inside in cases other than a case where the sample solution is to be put in. The sample solution 40 is liquid that is obtained after nucleic acid is extracted from samples collected from, for example, a nasal cavity, a pharynx, an oral cavity, an affected area, and the like of an examinee.

In the following description, a surface on which the inlet 12 is provided will be referred to as an upper surface of the test container 10 and a side corresponding to the bottom member 10B will be referred to as a lower surface of the test container 10. Here, an upper surface of the body member 10A is the same as the upper surface of the test container 10, a lower surface of the body member 10A is a surface that is in contact with an upper surface of the bottom member 10B, and a lower surface of the bottom member 10B is the same as the lower surface of the test container 10.

The liquid reservoir portion 13 is provided at a position facing the inlet 12 and stores the sample solution 40 dropped from the inlet 12. The shape of the liquid reservoir portion 13 is not particularly limited, and a columnar shape, a conical shape, a truncated conical shape, and the like can be arbitrarily selected as the shape of the liquid reservoir portion 13.

In this test container 10, the inlet 12 is formed of the opening of the tubular portion 14 that is formed to penetrate the body member 10A in a thickness direction and to protrude from the upper surface of the body member 10A, and the liquid reservoir portion 13 is formed by an inner side portion of the tubular portion 14 in the body member 10A and the bottom member 10B. The liquid reservoir portion 13 is a space for storing the sample solution 40, which is put in from the inlet 12, in front of the first chamber 15.

The first chamber 15 is a chamber that can store liquid, and is a chamber in which the sample solution 40 to be fed from the inlet 12 via the liquid reservoir portion 13 is stored. The first chamber 15 is formed by the recessed portion, which is provided on the lower surface of the body member 10A, and the bottom member 10B.

The first chamber 15 is, for example, a chamber to be subjected to pretreatment for a test, and is subjected to heating treatment performed by a first heating unit 120 in the test device 100 (see Fig. 14) to be described later.

The second chamber 16 is a chamber that can store liquid, and is a chamber in which the sample solution 40 to be fed from the first chamber 15 is stored. In this example, the sample solution 40 is fed to the second chamber 16 from the first chamber 15 via the liquid storage portion 24 of the valve 20 to be described later. The second chamber 16 is formed by the recessed portion, which is provided on the lower surface of the body member 10A, and the bottom member 10B.

The second chamber 16 is, for example, a chamber to be subjected to a reaction and detection processing for a test, and is subjected to heating treatment performed by a second heating unit 122 in the test device 100 (see Fig. 14) to be described later.

As shown in Fig. 2A, the valve 20 includes the liquid storage portion 24 that temporarily stores the sample solution 40 to be fed from the first chamber 15 to the second chamber 16. The valve 20 is a valve that switches a connection state between the first and second chambers 15 and 16 and the liquid storage portion 24 to any one of a first state, a second state, or a third state to be described below. The first state is a state where the first chamber 15 and the liquid storage portion 24 communicate with each other and the second chamber 16 and the liquid storage portion 24 do not communicate with each other. The second state is a state where the second chamber 16 and the liquid storage portion 24 communicate with each other and the first chamber 15 and the liquid storage portion 24 do not communicate with each other. The third state is a state where both the first chamber 15 and the second chamber 16 do not communicate with the liquid storage portion 24.

Fig. 2A shows the state where both the first chamber 15 and the second chamber 16 do not communicate with the liquid storage portion 24. In the schematic view showing the flow channel structure of Fig. 2B, × shown on the flow channel 18b schematically shows that the first chamber 15 and the liquid storage portion 24 are in a closed state where the first chamber 15 and the liquid storage portion 24 do not communicate with each other. Likewise, × shown on the flow channel 18c schematically shows that the liquid storage portion 24 and the second chamber 16 are in a closed state where the liquid storage portion 24 and the second chamber 16 do not communicate with each other. In the following drawings as well, × shown on the flow channel means that the chambers connected to each other via the flow channel do not communicate with each other.

The valve 20 of the test container 10 includes a rotating member 25 disposed in a tubular portion 22 consisting of a cylindrical recessed portion that is provided on the upper surface of the body member 10A and a portion that surrounds the recessed portion and protrudes from the upper surface of the body member 10A. The rotating member 25 is a cylindrical member having an outer diameter slightly smaller than an inner diameter of the tubular portion 22, and is rotatably installed in the tubular portion 22. The liquid storage portion 24 is provided in the rotating member 25, and one hole 24a communicating with the liquid storage portion 24 is provided on a bottom surface of the rotating member 25. A bottom surface of the tubular portion 22 includes a hole 22a that communicates with the flow channel 18b and a hole 22b that communicates with the flow channel 18c. Each of the holes 22a and 22b is provided with an O-ring 26. An upper surface of the rotating member 25 is provided with a knob 25a that is used to rotate the rotating member 25.

The O-ring 26 has a function to prevent leakage from a connection portion between the hole 24a and the hole 22a or the hole 22b in a case where the hole 24a of the bottom surface of the rotating member 25 and any one of the hole 22a or the hole 22b of the bottom surface of the tubular portion 22 coincide with each other and the liquid storage portion 24 communicates with the flow channel 18b or the flow channel 18c. A publicly known sealing member, such as a packing or a gasket, can be used as a sealing element for preventing leakage without particularly limited in addition to the O-ring 26. For example, rubber materials, such as nitrile rubber, urethane rubber, silicon rubber, and fluororubber, or publicly known elastomer, such as olefin and urethane, can be used as a material of the sealing element. Further, the sealing portion may be integrally molded with the rotating member 25 or the tubular portion 22 of the body member 10A.

The rotating member 25 can be rotated to set a state where the hole 24a of the bottom surface of the rotating member 25 and the hole 22a of the bottom surface of the tubular portion 22 coincide with each other, a state where the hole 24a and the hole 22b coincide with each other, and a state where the hole 24a coincides with neither of the hole 22a and the hole 22b.

Each of the flow channels 18a to 18c is formed by the recessed portion, which is provided on the lower surface of the body member 10A, and the bottom member 10B. The flow channel 18a is a flow channel that connects the liquid reservoir portion 13 and the first chamber 15. The flow channel 18b is a flow channel that connects the first chamber 15 and the liquid storage portion 24 of the valve 20. The flow channel 18b communicates with the hole 22a of the bottom surface of the tubular portion 22 of the valve 20. The flow channel 18c is a flow channel that connects the second chamber 16 and the liquid storage portion 24 of the valve 20. The flow channel 18c communicates with the hole 22b of the bottom surface of the tubular portion 22. A size of the cross section of each of the flow channels 18a to 18c is, for example, about 0.5 mm to 1 mm in width and 0.5 mm in depth.

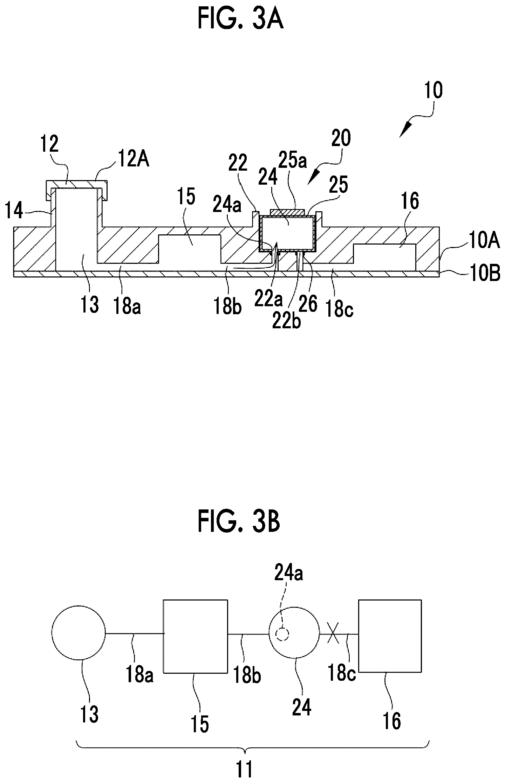

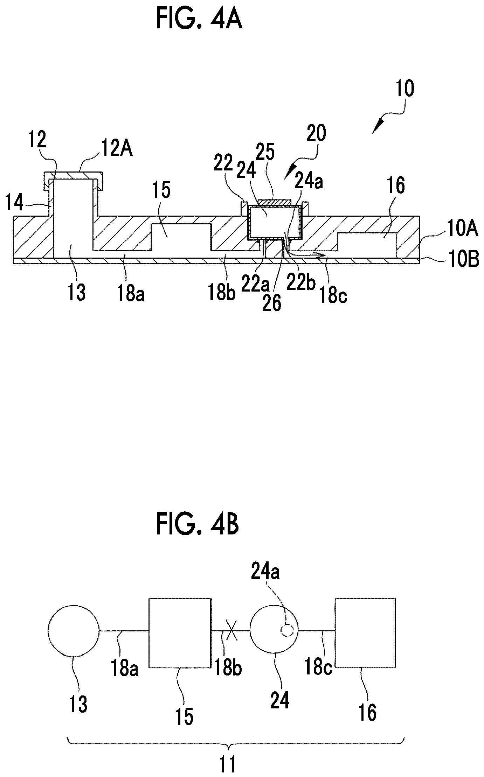

Figs. 3A and 3B and Figs. 4A and 4B are diagrams illustrating the first state and the second state that are switched by the valve 20. Figs. 3A and 3B are diagrams illustrating the test container 10 that is in the first state, in which Fig. 3A is a schematic cross-sectional view of the test container 10 and Fig. 3B is a schematic plan view of the flow channel structure 11. Figs. 4A and 4B are diagrams illustrating the test container 10 that is in the second state, in which Fig. 4A is a schematic cross-sectional view of the test container 10 and Fig. 4B is a schematic plan view of the flow channel structure 11.

In Figs. 3A and 3B, the hole 24a of the bottom surface of the rotating member 25 coincides with the hole 22a of the bottom surface of the tubular portion 22. In this case, the test container 10 is in the first state where the first chamber 15 and the liquid storage portion 24 communicate with each other and the second chamber 16 and the liquid storage portion 24 do not communicate with each other.

As shown in Figs. 4A and 4B, the hole 24a of the bottom surface of the rotating member 25 coincides with the hole 22b of the bottom surface of the tubular portion 22. In this case, the test container 10 is in the second state where the second chamber 16 and the liquid storage portion 24 communicate with each other and the first chamber 15 and the liquid storage portion 24 do not communicate with each other.

A state where the hole 24a of the bottom surface of the rotating member 25 coincides with neither of the hole 22a and the hole 22b of the bottom surface of the tubular portion 22 as shown in Figs. 2A and 2B is the third state where the liquid storage portion 24 does not communicate with both of the first chamber 15 and the second chamber 16.

As described above, the valve 20 is a rotary valve that can switch between the first state, the second state, and the third state in a case where the rotating member 25 is rotated in the tubular portion 22 of the body member 10A. The valve 20 may be any valve as long as the first state to the third state can be realized, and is not limited to the rotary valve having the above-described structure.

As shown in Figs. 2A and 2B, the sealing part 19 is provided in a part of the flow channel structure 11 to prevent the back flow of the sample solution 40 after the sample solution 40 is fed from the liquid reservoir portion 13 to the first chamber 15.

In the example shown in Figs. 2A and 2B, the sealing part 19 is a hydrophobic substance having a specific gravity lower than that of the sample solution 40 (hereinafter, referred to as a hydrophobic substance 19).

The hydrophobic substance 19 is a low-polarity solvent or a thermoplastic substance. The hydrophobic substance 19 may be a substance that is liquid at room temperature or a substance that is solid at room temperature but becomes liquid by being heated. Examples of the low-polarity solvent include silicone oil, a hydrocarbon-based solvent, a fluorine-based solvent, carbon tetrachloride, and the like. Here, low-polarity means a polarity to such an extent that the solvent does not mix with the sample solution 40. Examples of the thermoplastic substance include various waxes, adhesives, and the like.

In the test container 10 having this configuration, the hydrophobic substance 19 is provided in the liquid reservoir portion 13 or the first chamber 15. In the example shown in Figs. 2A and 2B, the hydrophobic substance 19 is provided in the first chamber 15.

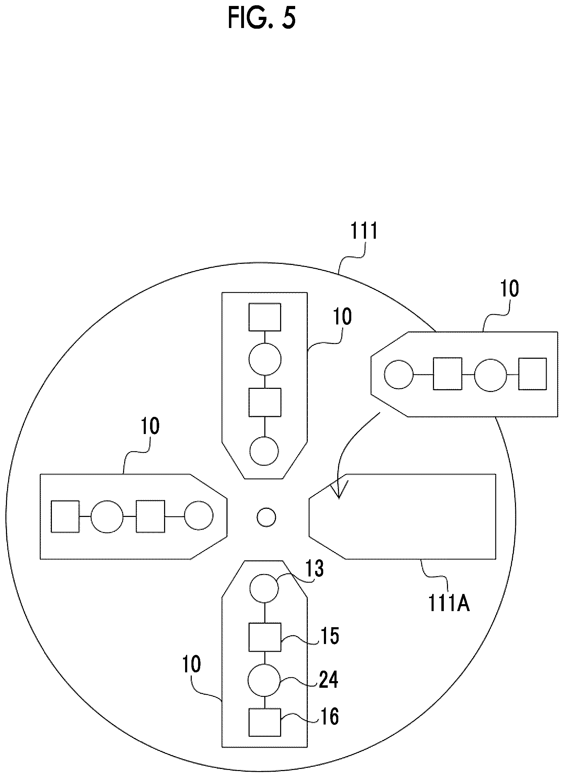

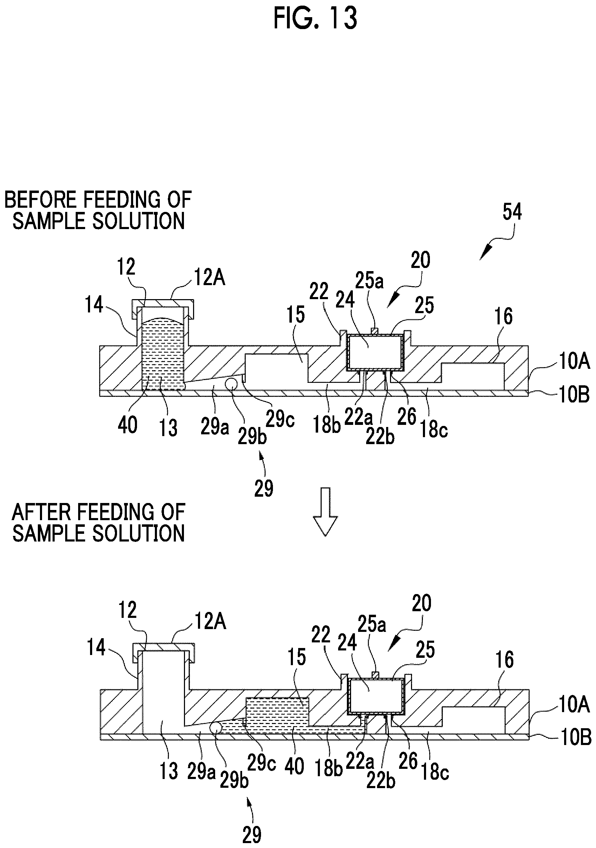

For example, the test container 10 is set on a loading portion 111A of a disk-shaped support 111 shown in Fig. 5 and is rotated about an axis A in the test device 100 (see Fig. 13) including the centrifuge 110. The test container 10 is set on the centrifuge 110 such that the liquid reservoir portion 13, the first chamber 15, the liquid storage portion 24, and the second chamber 16 are arranged in this order from an inner diameter side close to the axis A serving as a center of rotation.

The sample solution 40 put in from the inlet 12 is fed from the liquid reservoir portion 13 to the first chamber 15, is fed from the first chamber 15 to the liquid storage portion 24, and is fed from the liquid storage portion 24 to the second chamber 16, respectively, by a centrifugal force caused by the rotation of the centrifuge 110.

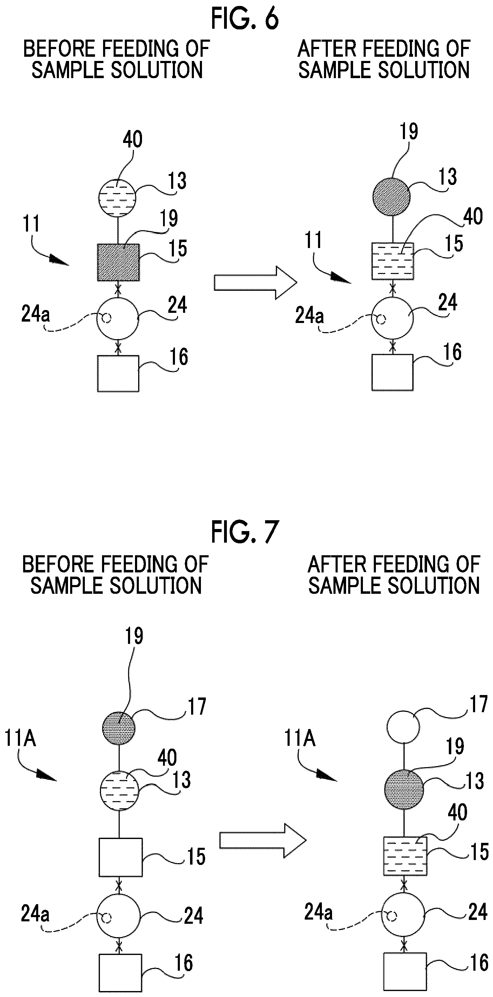

Here, a method of feeding the sample solution 40 from the liquid reservoir portion 13 to the first chamber 15 and the function of the hydrophobic substance 19 will be described with reference to Fig. 6. Fig. 6 shows the states of the flow channel structure 11 before and after the sample solution 40 is fed from the liquid reservoir portion 13 to the first chamber 15.

The sample solution 40 is put in in a state where the first chamber 15 and the liquid storage portion 24 of the valve 20 are caused not to communicate with each other by the valve 20. In an example shown in Fig. 6, the third state where neither of the first chamber 15 and the second chamber 16 does not communicate with the liquid storage portion 24 is set but the second state may be set.

As shown in a left diagram of Fig. 6, a state where the sample solution 40 is stored in the liquid reservoir portion 13 is set immediately after the sample solution 40 is put in from the inlet 12. At this point in time, the hydrophobic substance 19 is stored in the first chamber 15. In a case where the test container 10 is rotated by the centrifuge in this state, the sample solution 40 having a relatively high specific gravity is moved to an outer diameter side away from the center and the hydrophobic substance 19 having a relatively low specific gravity is moved to the inner diameter side as shown in a right diagram of Fig. 6. As a result, the sample solution 40 is fed from the liquid reservoir portion 13 to the first chamber 15.

As described above, the test container 10 according to the present embodiment comprises the sealing part 19 (in this example, the hydrophobic substance 19) that prevents the back flow of the sample solution 40 after the sample solution 40 is fed from the liquid reservoir portion 13 to the first chamber 15. According to such a configuration, in a case where the first chamber 15 is heated for pretreatment of the sample solution 40, for example, in a case where the first chamber 15 is heated to a high temperature equal to or higher than 90°C in a state where the sample solution 40 is stored in the first chamber 15, it is possible to suppress the back flow of the sample solution 40 to the liquid reservoir portion 13.

In a case where the hydrophobic substance 19 is provided as the sealing part as in the present embodiment, the sample solution 40 and the hydrophobic substance 19 are exchanged with each other after the sample solution 40 is fed to the first chamber 15. As a result, a state where the hydrophobic substance 19 is stored in the liquid reservoir portion 13 is set. Since the hydrophobic substance 19 is stored in the liquid reservoir portion 13, it is possible to suppress the back flow of the vaporized sample solution 40 to the liquid reservoir portion 13 as compared to a case where the hydrophobic substance 19 is not provided and only air is present in the liquid reservoir portion 13. Since it is possible to suppress the back flow of the sample solution 40 from the first chamber 15 to the liquid reservoir portion 13 without providing a mechanical structure, the sealing part can be realized in a very simple manner and at a low cost.

It is preferable that the hydrophobic substance 19 is provided by volume enough to fill the liquid reservoir portion 13. In a case where the hydrophobic substance 19 fills the liquid reservoir portion 13 after the sample solution 40 is fed to the first chamber 15, it is possible to more effectively suppress the back flow of the sample solution 40 from the first chamber 15 to the liquid reservoir portion 13.

Since the hydrophobic substance 19 does not mix with the sample solution 40 in a case where the hydrophobic substance 19 is a low-polarity solvent, it is possible to suppress the back flow of the sample solution 40 to the liquid reservoir portion 13.

In a case where the hydrophobic substance 19 is a thermoplastic substance, the hydrophobic substance 19 is liquefied and the sample solution 40 is fed, so that the hydrophobic substance 19 is fed to the liquid reservoir portion 13 and is solidified by being cooled in the liquid reservoir portion 13. Accordingly, it is possible to effectively suppress the back flow of the sample solution 40 to the liquid reservoir portion 13.

In the above-described embodiment, the hydrophobic substance 19 is provided in the liquid reservoir portion 13 or the first chamber 15 before the sample solution 40 is put in. A portion in which the hydrophobic substance 19 is provided is not limited thereto. As shown in Fig. 7, a flow channel structure 11A of a modification example may comprise a hydrophobic substance storage portion 17 that is provided on a side closer to a rotation axis of the centrifuge than the liquid reservoir portion 13, and the hydrophobic substance 19 may be stored in the hydrophobic substance storage portion 17.

In a test container comprising the flow channel structure 11A of the modification example, the hydrophobic substance 19 is stored in the hydrophobic substance storage portion 17 in a state where the sample solution 40 is put in from the inlet 12 and stored in the liquid reservoir portion 13. In a case where the test container is rotated by the centrifuge in this state, the sample solution 40 is fed from the liquid reservoir portion 13 to the first chamber 15 and the hydrophobic substance 19 is fed to the liquid reservoir portion 13 as shown in the right diagram of Fig. 7.

Even in a case where the hydrophobic substance 19 is provided in the hydrophobic substance storage portion 17 provided on a side closer to a rotation axis of the centrifuge than the liquid reservoir portion 13 as described above, the hydrophobic substance 19 is stored in the liquid reservoir portion 13 after the sample solution 40 is fed. Therefore, it is possible to suppress the back flow of the sample solution 40 to the liquid reservoir portion 13.

In a case where a volume of the liquid reservoir portion 13 is denoted by V1, a volume of the first chamber 15 is denoted by V2, a volume of the liquid storage portion 24 is denoted by V3, and a volume of the second chamber 16 is denoted by V4 in the flow channel structure 11 (or the flow channel structure 11A), it is preferable that a relationship of "V1 ≥ V2 ≥ V3 ≥ V4" is satisfied. That is, it is preferable that the volumes of the adjacent chambers are equal to each other or the chamber disposed closer to the outer diameter side has a smaller volume among the plurality of chambers arranged from the inner diameter side toward the outer diameter side of the centrifuge.

For example, the volume V1 of the liquid reservoir portion 13 exceeds 100 µL and is 200 µL or less, the volume V2 of the first chamber 15 is 100 µL, the volume V3 of the liquid storage portion 24 is 50 µL, and the volume V4 of the second chamber 16 is 30 µL.

In a case where the volume V1 of the liquid reservoir portion 13 is larger than the volume of the first chamber 15, the liquid reservoir portion 13 can store the sample solution 40 more than the volume V2 of the first chamber 15. Further, in a case where a relationship between the volumes of the plurality of chambers arranged from the inner diameter side toward the outer diameter side of the centrifuge satisfies "V1 ≥ V2 ≥ V3 ≥ V4", the chamber disposed on the outer diameter side (that is, the downstream side) can be filled with the sample solution 40 in a case where the sample solution 40 is fed from the chamber disposed on the inner diameter side of the centrifuge to the chamber disposed on the outer diameter side. For this reason, it is possible to suppress the occurrence of the shortage of the sample solution 40 in the second chamber 16 finally used for the detection.

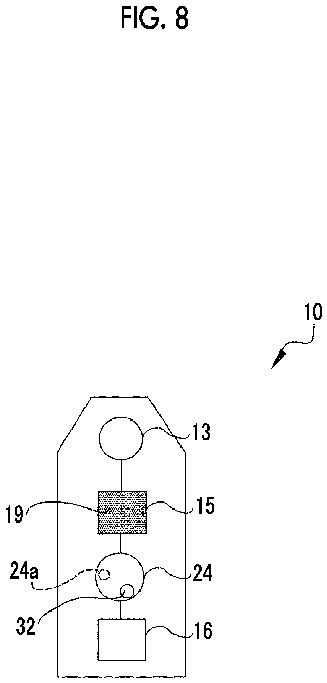

As shown in Fig. 8, the test container 10 may comprise a reagent 32 that reacts with the sample solution 40 in the flow channel structure 11. For example, in a case where the test container 10 is used to detect nucleic acid, the reagent 32 is a reagent that contains an amplification reagent for amplifying a specific nucleic acid sequence and a fluorescent probe for determining a nucleic acid sequence. The reagent 32 may be provided in any of the liquid reservoir portion 13, the first chamber 15, the liquid storage portion 24, and the second chamber 16. The reagent 32 is preferably provided in the first chamber 15 or the liquid storage portion 24, and particularly preferably provided in the liquid storage portion 24.

A stirring rod for promoting the mixing of the sample solution 40 and the reagent 32 may be included in the chamber in which the reagent 32 is stored or a chamber that is provided on the downstream side thereof.

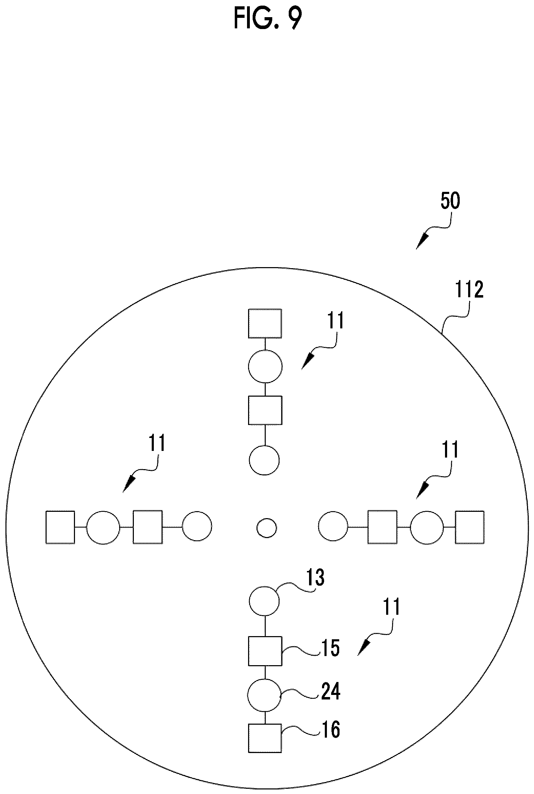

The test container 10 according to the embodiment includes one flow channel structure 11 from the inlet 12 to the second chamber 16. As a test container 50 of a modification example, one test container 50 may comprise a plurality of flow channel structures 11 as shown in Fig. 9. The test container 50 shown in Fig. 9 comprises a disk-shaped body member and a bottom member, and comprises four flow channel structures 11 that are disposed radially around a rotation axis A. Of course, the number of flow channel structures 11 provided in one test container 50 is not limited to four.

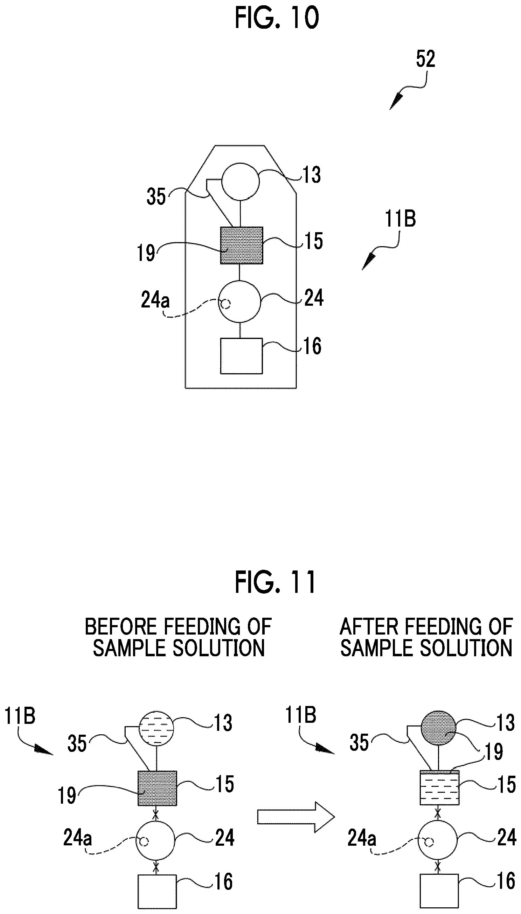

Further, as in a test container 52 of a modification example shown in Fig. 10, a flow channel structure 11B may comprise a vent flow channel 35. In this example, the vent flow channel 35 is a flow channel that connects the first chamber 15 and the liquid reservoir portion 13. The vent flow channel 35 is a flow channel through which gas passes. Since the vent flow channel 35 is provided, the sample solution 40 is easily fed from the liquid reservoir portion 13 to the first chamber 15. As a result, the sample solution 40 can be efficiently fed. On the other hand, in a case where the vent flow channel 35 is provided and the first chamber 15 is heated to a high temperature for pretreatment after the sample solution 40 is fed to the first chamber 15, the vaporized sample solution 40 may return to the liquid reservoir portion 13 via the vent flow channel 35. However, since the hydrophobic substance is stored in the liquid reservoir portion 13 in the present embodiment, the back flow of the sample solution 40 from the vent flow channel 35 can also be suppressed.

Fig. 11 shows states of the flow channel structure 11B of the test container 52 before and after the sample solution is fed. In a case where, after the sample solution 40 is fed to the first chamber 15, the hydrophobic substance 19 fills the liquid reservoir portion 13, a part of the hydrophobic substance 19 remains in the first chamber 15, and an entrance of the vent flow channel 35 of the first chamber 15 is blocked by the hydrophobic substance 19 as shown in Fig. 11, it is possible to more effectively prevent the back flow of the sample solution 40 to the vent flow channel 35.

However, considering that the vent flow channel 35 may not be completely blocked, it is more preferable that the vent flow channel is not provided as in the test container 10.

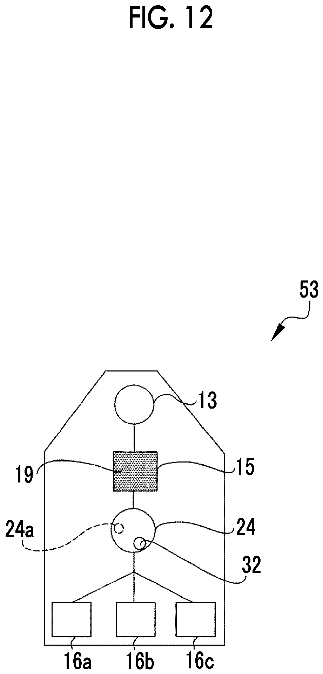

Further, a plurality of second chambers 16a to 16c may be provided as in a test container 53 of a modification example shown in Fig. 12. In a case where the plurality of second chambers 16a to 16c are provided, the same test is performed for one sample using the plurality of second chambers 16a to 16c, so that test accuracy can be improved. Furthermore, in a case where the plurality of second chambers 16a to 16c are provided, a plurality of test target substances can be tested in parallel for one sample. The first second chamber 16a is used to test for influenza, the second second chamber 16b is used to test for a novel coronavirus, and the third second chamber 16c is used to test for a respiratory syncytial (RS) virus. Although an example in which three second chambers 16a to 16c are provided in the example shown in Fig. 12, the number of second chambers is not limited. However, in a case where a plurality of second chambers 16a to 16c are provided, it is desirable that the total volume of all the second chambers 16a to 16c is smaller than the volume of the liquid storage portion 24 of the valve 20.

In the above-described embodiment, the hydrophobic substance 19 is provided as the sealing part. However, the sealing part has only to function to prevent the back flow of the sample solution 40 after the sample solution 40 is fed from the liquid reservoir portion 13 to the first chamber 15, and a structure such as a check valve may be provided between the liquid reservoir portion 13 and the first chamber 15.

Fig. 13 is schematic cross-sectional views of a test container 54 of a modification example. Further, Fig. 13 shows states of a flow channel structure 11 before and after the sample solution 40 is fed from a liquid reservoir portion 13 to a first chamber 15.

In the test container 54, a flow channel 29a that connects the liquid reservoir portion 13 and the first chamber 15 is formed in a tapered shape in which a diameter gradually increases from the liquid reservoir portion 13 toward the first chamber 15. A sphere 29b is provided in the flow channel 29a. A diameter of the sphere 29b is larger than the diameter of a portion of the flow channel 29a closest to the liquid reservoir portion 13. A stopper 29c that restricts the movement of the sphere 29b to the first chamber 15 is provided at a portion of the flow channel 29a close to the first chamber 15. In this example, a sealing part 29 that prevents the back flow of the sample solution 40 after the sample solution 40 is fed from the liquid reservoir portion 13 to the first chamber 15 is formed by the flow channel 29a, the sphere 29b, and the stopper 29c.

The sphere 29b of the test container 54 is disposed to be movable in the flow channel 29a before the sample solution is fed. In an upper diagram of Fig. 13, the sample solution 40 is put in from the inlet 12 and is stored in the liquid reservoir portion 13. As in the case of the embodiment described above, the test container 54 is rotated by the centrifuge in a state where a portion between the first chamber 15 and the liquid storage portion 24 is closed. Accordingly, as shown in a lower diagram of Fig. 13, the sample solution 40 is fed to the first chamber 15 by a centrifugal force. In a case where the first chamber 15 is filled with the sample solution 40, the sphere 29b is gradually pushed toward the liquid reservoir portion 13 by the sample solution 40 and is moved to a portion that has a diameter equal to the diameter of the sphere 29b. Accordingly, the sphere 29b functions as a check valve that blocks the flow channel 29a.

According to this configuration, according to such a configuration, in a case where the first chamber 15 is heated for pretreatment of the sample solution 40, for example, in a case where the first chamber 15 is heated to a high temperature of 90°C or higher in a state where the sample solution 40 is stored in the first chamber 15, the sphere 29b is further pushed toward the liquid reservoir portion 13 and seals the flow channel 29a as the sample solution 40 is vaporized and pressure in the first chamber 15 is increased. Therefore, it is possible to suppress the back flow of the sample solution 40 to the liquid reservoir portion 13.

Further, a configuration in which a bottom member 10B forming a bottom surface of a flow channel 18c, which connects the liquid reservoir portion 13 and the first chamber 15, is formed of a film and the bottom member 10B can be pressed from the outside to block the flow channel 18c may be provided as the sealing part.

Each of the flow channel structures 11, 11A, and 11B of the test containers 10, 50, 52, and 54 described above may further comprise a deformable chamber that communicates with the second chamber 16. In a case where the deformable chamber is deformed by being pressed from the outside or the like, the deformable chamber and the second chamber 16 communicating with the deformable chamber can be pressurized. In a case where the second chamber 16 is pressurized, a temperature rising property and a cooling property of liquid stored in the second chamber 16 can be improved. Further, in a case where the second chamber 16 is pressurized, the vaporization of the liquid stored therein can be suppressed during heating.

### "Test device"

The test device 100 into which the test containers 10 are loaded and which is used to perform a test will be described with reference to Fig. 14. Fig. 14 is a diagram showing a schematic configuration of the test device 100. The test device 100 is, for example, a nucleic acid test device.

The test device 100 comprises a centrifuge 110, a first heating unit 120, a second heating unit 122, a valve control unit 124, and a detection unit 126.

The centrifuge 110 comprises a seat 112 that is provided with a rotation mechanism such as a motor, a disk-shaped support 111 that is provided with loading portions 111A (see Fig. 5) into which the test containers 10 are to be loaded and forms a rotary table, and a rotary coupler 114 that is connected to a shaft of the rotation mechanism and supports the disk-shaped support 111 to allow the disk-shaped support 111 to be rotatable. In a case where the rotation mechanism is driven, the disk-shaped support 111 is rotated about an axis A. The feeding of liquid is realized in the flow channel structure, which is provided in the test container 10, by a centrifugal force accompanying this rotation. In the example shown in Fig. 13, the disk-shaped support 111 includes pressers 111B that press the test containers 10 set on the loading portions 111A.

The first heating unit 120 is provided to be movable between a position where the first heating unit 120 is in contact with a bottom surface of the first chamber 15 of the test container 10 set on the disk-shaped support 111 and a position where the first heating unit 120 is separated from the bottom surface of the first chamber 15. While the disk-shaped support 111 is rotating, the first heating unit 120 is disposed at a position where the first heating unit 120 is separated from the bottom surface of the first chamber 15. In a state where the disk-shaped support 111 is stopped, the first heating unit 120 is disposed at a position where the first heating unit 120 is in contact with the bottom surface of the first chamber 15. The first heating unit 120 heats liquid stored in the first chamber 15. Here, the liquid to be stored in the first chamber 15 is the sample solution 40. The first heating unit 120 heats the sample solution 40 to a high temperature of, for example, 90°C or higher for pretreatment.

The second heating unit 122 is provided to be movable between a position where the second heating unit 122 is in contact with a bottom surface of the second chamber 16 of the test container 10 set on the disk-shaped support 111 and a position where the second heating unit 122 is separated from the bottom surface of the second chamber 16. While the disk-shaped support 111 is rotating, the second heating unit 122 is disposed at a position where the second heating unit 122 is separated from the bottom surface of the second chamber 16. In a state where the disk-shaped support 111 is stopped, the second heating unit 122 is disposed at a position where the second heating unit 122 is in contact with the bottom surface of the second chamber 16. The second heating unit 122 heats liquid stored in the second chamber 16. Here, the liquid to be stored in the second chamber 16 is, for example, a liquid mixture of the sample solution 40 and the reagent 32. The second heating unit 122 heats the liquid mixture of the sample solution 40 and the reagent 32 to promote the amplification of a nucleic acid.

The second heating unit 122 comprises a Peltier element or the like and is adapted to be capable of controlling a temperature, and performs a temperature cycle in a nucleic acid amplification step. On the other hand, the first heating unit 120 does not require the temperature cycle performed by the second heating unit 122, and is formed of, for example, a heater. A publicly known heating mechanism can be used as a heating mechanism used for each of the first heating unit 120 and the second heating unit 122, and the heating mechanism is not particularly limited.

The valve control unit 124 comprises a mechanism that controls the valve 20 to switch a communication state between the first chamber 15, the second chamber 16, and the liquid storage portion 24 to the first state, the second state, and the third state. The valve control unit 124 comprises, for example, a grip part that grips the knob 25a of the rotating member 25, a rotation mechanism that rotates the grip part in a state where the grip part grips the knob 25a, and a processor that drives the rotation mechanism as necessary in a test processing step.

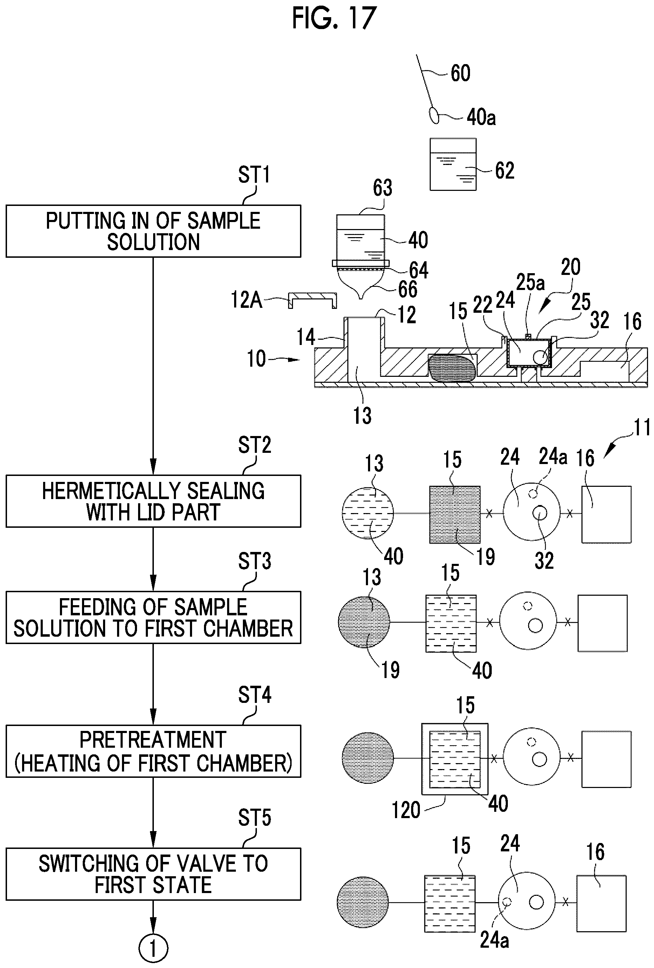

The detection unit 126 detects whether or not an object to be detected is contained in the sample solution 40 in the second chamber 16. As shown in Fig. 17, the detection unit 126 comprises a light source 126a, a wavelength selective filter 126b, and a photodetector 126c. The detection unit 126 is disposed above the second chamber 16 of the test container 10. Detection using the detection unit 126 is also performed in a state where rotation using the centrifuge 110 is stopped. The light source 126a emits excitation light L1 having a specific wavelength into the second chamber 16 via the wavelength selective filter 126b. The photodetector 126c detects fluorescence L2 that is excited by the excitation light L1 and is generated from a fluorescent probe. The excitation light L1 is selected according to an excitation wavelength of the fluorescent probe. Further, the detection unit 126 may include a filter that adjusts intensity or the amount of light, a lens or an optical system that is used to converge the excitation light L1 or to condense the fluorescence L2 generated from the detection probe to the photodetector 126c, or the like, as necessary.

An LED, a laser, or the like is used as the light source 126a. The wavelength selective filter 126b is a filter that transmits only light, which has a wavelength corresponding to the excitation wavelength of the probe, of the light emitted from the light source 126a. For example, a photodiode, a photomultiplier, or the like is applied as the photodetector 126c.

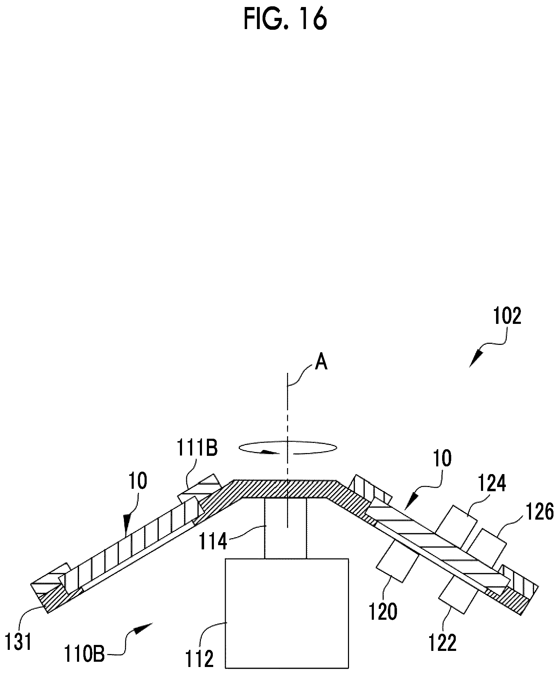

A test device 101 shown in Fig. 15, a test device 102 shown in Fig. 16, or the like can also be applied as a test device in which the test container 10 is used, in addition to the test device 100 shown in Fig. 14 described above. The same components as those of the test device 100 shown in Fig. 14 will be denoted in Figs. 15 and 16 by the same reference numerals, and the detailed description thereof will be omitted.

The test device 101 and the test device 102 comprise centrifuges 110A and 110B, which have structures different from the structure of the centrifuge 110 of the test device 100, respectively.

The test device 101 shown in Fig. 15 is adapted such that the centrifuge 110A rotates a disk-shaped support 111 in a state where a surface of the disk-shaped support 111 is inclined from a horizontal plane. A rotation axis A of the centrifuge 110A is inclined with respect to a direction perpendicular to the horizontal plane.

In the test device 101 shown in Fig. 16, the centrifuge 110B comprises an umbrella-shaped support 131 instead of the disk-shaped support 111. The umbrella-shaped support 131 comprises a horizontal portion that is supported by a rotary coupler 114 and an inclined portion which is provided to surround the horizontal portion and of which an outer peripheral side is positioned below the horizontal portion in a vertical direction, and the test containers 10 are set on the inclined portion.

The test container 10 can also be applied in the case of a centrifuge having any configuration of various centrifuges 110A, 110B, and 110C shown in Figs. 14 to 16.

In the above description, the test devices 100, 101, and 102 have been described as nucleic acid test devices used for a nucleic acid test accompanied by nucleic acid amplification treatment. In a case where the first heating unit 120, the second heating unit 122, and the detection unit 126 of each of the test devices 100, 101, and 102 are adapted to be suitable for various tests, the test devices 100, 101, and 102 can be used for various tests other than a nucleic acid test.

### "Nucleic acid test method"

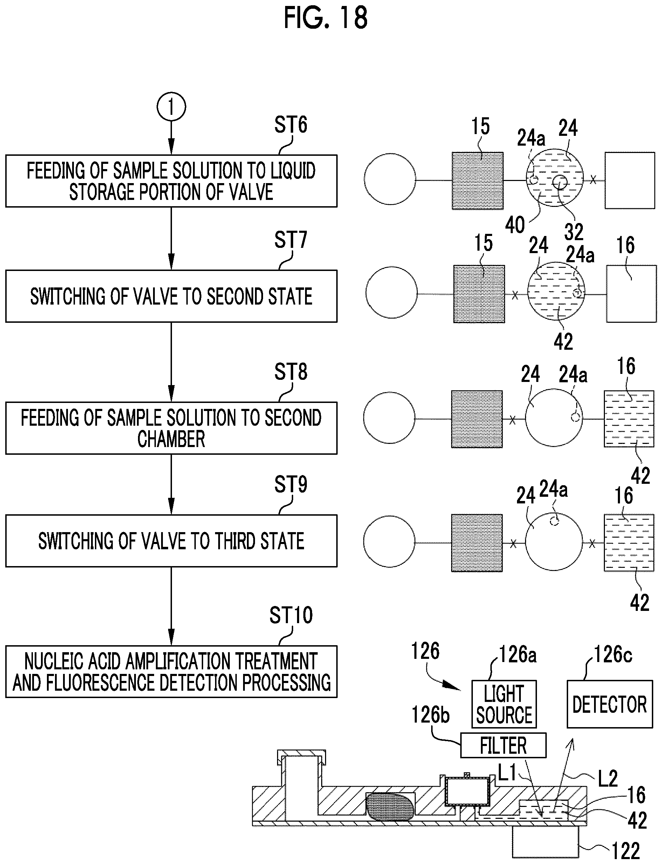

A nucleic acid test method according to an embodiment using the test container 10 according to the above-described embodiment will be described with reference to Figs. 17 and 18. Here, a test container 10 (see Fig. 8) in which the hydrophobic substance 19 as a sealing part is provided in the first chamber 15 and the reagent 32 for a nucleic acid test is provided in the liquid storage portion 24 is used.

First, as shown in Fig. 17, as a preparation, a sample 40a collected from a living body using a collection tool 60 such as a swab is immersed in an extraction solution 62 containing a surfactant and proteinase K (ProK) to perform extraction of nucleic acid and treatment of impurities. Hereinafter, liquid containing the sample 40a, which is mixed with the extraction solution 62 and from which nucleic acid has been extracted in the extraction solution 62, is referred to as a sample solution 40.

The sample 40a is collected from, for example, a nasal cavity, a pharynx, the inside of an oral cavity, or an affected area of an examinee using a collection tool. Alternatively, the sample 40a is body fluid, such as a washing solution in a nasal cavity, a pharynx, or an oral cavity, saliva, urine, or blood. A publicly known nucleic acid extraction method can be used as a nucleic acid extraction method without particular limitation. Examples of the nucleic acid extraction method include a method using a surfactant or a chaotropic substance and a method of applying physical shear, such as an ultrasonic wave or a bead mill.

In an initial state before the sample solution 40 is put into the test container 10, the valve 20 is set to the second state or the third state where at least the first chamber 15 and the liquid storage portion 24 do not communicate with each other. In the present embodiment, the valve 20 is set to the third state where neither of the first chamber 15 and the second chamber 16 communicates with the liquid storage portion 24. In the test, first, a dropping cap 66 provided with a filter 64 for removing coarse impurities is attached to a container 63 that stores the sample solution 40 containing the sample 40a from which nucleic acid has been extracted in the extraction solution as described above while the valve 20 remains in the initial state, and the sample solution 40 filtered by the filter 64 is put in from the inlet 12 of the test container 10 (Step ST1). The sample solution 40 is stored in the liquid reservoir portion 13.

After the sample solution 40 is completely put in, the inlet 12 is closed by the lid part 12A and an inner space of the test container 10 is hermetically sealed (Step ST2). The test container 10 is loaded into the test device 100 in a state where the sample solution 40 is put in and the inlet 12 is closed by the lid part 12A. The following processing is performed in the test device 100.

The test container 10 is rotated by the centrifuge 110 of the test device 100, and the sample solution 40 is fed from the liquid reservoir portion 13 to the first chamber 15 (Step ST3). The sample solution 40 is fed to the first chamber 15, and the hydrophobic substance 19 stored in the first chamber 15 is moved to the liquid reservoir portion 13.

In a state where the back flow of the sample solution 40 from the first chamber 15 to the liquid reservoir portion 13 is prevented by the hydrophobic substance 19, the first chamber 15 is heated by the first heating unit 120 to perform the pretreatment of the sample solution 40 using heating treatment (Step ST4). The ProK is deactivated by the heating treatment. The heating temperature is, for example, preferably 80°C or higher, more preferably 85°C or higher, and still more preferably 90°C or higher. An upper limit temperature is preferably 100°C or lower and more preferably 95°C or lower from the viewpoint of vaporization of the sample solution.

After the pretreatment, the valve 20 is switched to the first state by the valve control unit 124 (see Fig. 14) (Step ST5). That is, the first chamber 15 and the liquid storage portion 24 communicate with each other, and the liquid storage portion 24 and the second chamber 16 do not communicate with each other.

Next, the test container 10 is rotated by the centrifuge 110 of the test device 100 and the sample solution 40 is fed from the first chamber 15 to the liquid storage portion 24 of the valve 20 (Step ST6). The reagent 32 is stored in the liquid storage portion 24, and the fed sample solution 40 and the reagent 32 are mixed with each other in the liquid storage portion 24.

After the sample solution 40 and the reagent 32 are mixed with each other, the valve 20 is switched to the second state by the valve control unit 124 (see Fig. 14) (Step ST7). That is, the second chamber 16 and the liquid storage portion 24 communicate with each other, and the liquid storage portion 24 and the first chamber 15 do not communicate with each other.

Then, the test container 10 is rotated by the centrifuge 110 of the test device 100 and a liquid mixture 42 of the sample solution 40 and the reagent 32 is fed from the liquid storage portion 24 of the valve 20 to the second chamber 16 (Step ST8).

After the liquid mixture 42 is fed to the second chamber 16, the valve 20 is switched to the third state by the valve control unit 124 (see Fig. 14) (Step ST9). Here, at least the second chamber 16 and the liquid storage portion 24 do not communicate with each other. Neither of the first chamber 15 and the second chamber 16 does not communicate with the liquid storage portion 24 in this example, but it is sufficient as long as the second chamber 16 and the liquid storage portion 24 do not communicate with each other. Accordingly, the valve 20 may be switched to the first state.

Here, the liquid mixture 42 is subjected to nucleic acid amplification treatment and fluorescence detection in a state where the second chamber 16 and the liquid storage portion 24 do not communicate with each other (Step ST10).

The nucleic acid amplification treatment is treatment of heating the liquid mixture 42, which is stored in the second chamber 16, by the second heating unit 122 to amplify a specific nucleic acid sequence. A nucleic acid amplification treatment method is not limited, and for example, an RT-PCR method or a PCR method is used. In a case where a PCR method is used, a step of dissociating a double-stranded DNA into a single-stranded DNA at a high temperature (thermal denaturation step), a step of lowering a temperature and binding a primer to the single-stranded DNA (annealing step), and a step of newly synthesizing a double-stranded DNA by polymerase using the single-stranded DNA as a template (elongation step) are repeated. Examples of a temperature cycle of the thermal denaturation step, the annealing step, and the elongation step include 20 to 50 repetitions of one cycle of the thermal denaturation step performed for 1 minute at a temperature of 94°C, the annealing step performed for 1 minute at a temperature of 50 to 60°C, and the elongation step performed for 1 to 5 minutes at a temperature of 72°C. Further, the thermal denaturation step and the annealing step may be performed at one temperature. Examples of such a temperature cycle include 20 to 50 repetitions of one cycle of the thermal denaturation step and the annealing step performed for 1 minute at a temperature of 94°C and the elongation step performed for 1 minute at a temperature of 60°C. The temperature and time of the temperature cycle of the amplification step are not particularly limited and are arbitrarily selected depending on the performance of polymerase or a primer.

The fluorescence detection is performed by the detection unit 126 disposed above the second chamber 16. Excitation light L1 having a specific wavelength is emitted into the second chamber 16 from the light source 126a via the wavelength selective filter 126b. Fluorescence L2, which is excited by the excitation light L1 and is generated from a fluorescent probe, is detected by the photodetector 126c. For example, in a case where the nucleic acid amplification treatment is a PCR method, fluorescence detection is performed for each cycle of the temperature cycle and an amplification situation is monitored in real time.

In a case where a specific nucleic acid sequence is present in the sample solution 40, the nucleic acid sequence is amplified in the amplification step and a fluorescent probe labeled with this specific nucleic acid sequence is irradiated with the excitation light L1, so that the fluorescence L2 is detected. On the other hand, in a case where a specific nucleic acid sequence is not present in the sample solution 40, the fluorescence L2 is not detected even though the excitation light L1 is emitted. Accordingly, it is possible to determine whether or not a specific nucleic acid sequence is present.

According to the test method using the test container 10, the sample solution 40 fed from the liquid reservoir portion 13 to the first chamber 15 is subjected to pretreatment using high-temperature heating in the first chamber 15. However, since the sealing part (in this example, the hydrophobic substance 19) for preventing the back flow of the sample solution 40 to the liquid reservoir portion 13 is provided, the back flow of the sample solution 40 can be suppressed. Accordingly, since it is possible to suppress a reduction in the amount of sample solution 40 to be finally treated in the second chamber 16, it is possible to improve test accuracy.

In the test method using the test device 100, whether or not a nucleic acid is present is determined by a fluorescence method using a fluorescent probe. However, a method of determining whether or not a nucleic acid is present is not limited to the fluorescence method, and other detection methods, such as a nucleic acid chromatography method, a light scattering method, a sequencing method, and an electrochemical method, may be used. These methods can be realized by the appropriate change of the detection unit, and the test container may be provided with a reagent containing a probe corresponding to each detection method instead of the fluorescent probe.

In the above description, a nucleic acid test has been exemplified as a test using the test container 10, but the test container 10 can be applied not only to the nucleic acid test but also to other gene tests, microbiological tests, and the like.

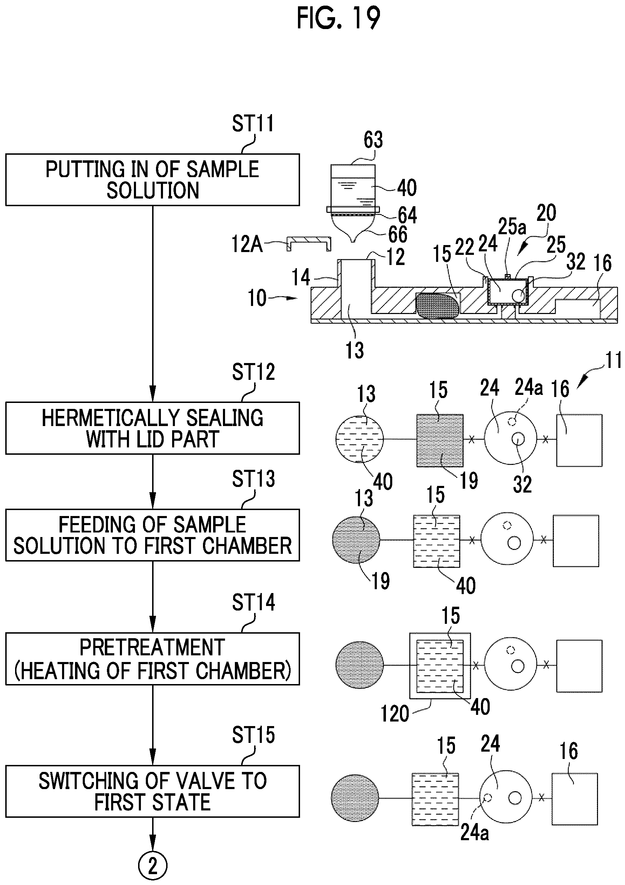

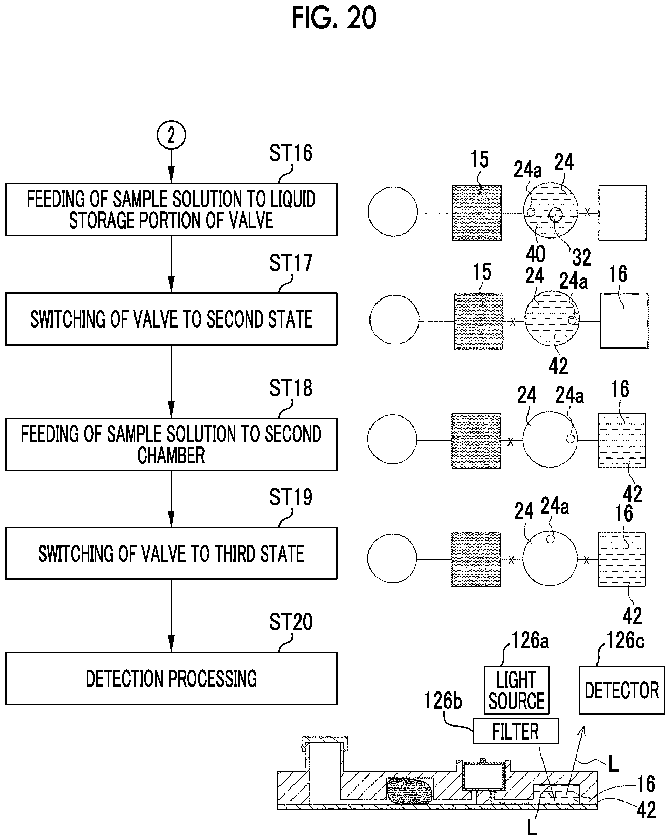

An example in which the test container 10 is used for a β-glucan test will be described as an example of the microbiological test with reference to Figs. 19 and 20. Here, a test container 10 (see Fig. 8) in which the hydrophobic substance 19 as a sealing part is provided in the first chamber 15 and a limulus amebocyte lysate (LAL) reagent is provided in the liquid storage portion 24 as the reagent 32 is used. In this example, a test device having the same configuration as the test device 100 can be used.

A sample and pretreatment liquid are mixed with each other to prepare a sample solution 40. A dropping cap 66 provided with a filter 64 is attached to a container 63 that stores the sample solution 40, and the sample solution 40 filtered by the filter 64 is put in from the inlet of the test container 10 (Step ST11). The sample solution 40 is stored in the liquid reservoir portion 13.

After the sample solution 40 is completely put in, the inlet 12 is closed by the lid part 12A and an inner space of the test container 10 is hermetically sealed (Step ST12). The test container 10 is loaded into the test device 100 in a state where the sample solution 40 is put in and the inlet 12 is closed by the lid part 12A. The following processing is performed in the test device 100.

The test container 10 is rotated by the centrifuge 110 of the test device 100, and the sample solution 40 is fed from the liquid reservoir portion 13 to the first chamber 15 (Step ST13). The sample solution 40 is fed to the first chamber 15, and the hydrophobic substance 19 stored in the first chamber 15 is moved to the liquid reservoir portion 13.

In a state where the back flow of the sample solution 40 from the first chamber 15 to the liquid reservoir portion 13 is prevented by the hydrophobic substance 19, the first chamber 15 is heated by the first heating unit 120 to perform temperature control for increasing the temperature of the sample solution 40 to 70°C for 10 minutes and cooling the sample solution 40 to 3°C (Step ST14). Pretreatment to be performed here is treatment of heating the sample solution 40 and stopping the reaction cascade of endotoxin.

After the pretreatment, the valve 20 is switched to the first state by the valve control unit 124 (see Fig. 14) (Step ST15). That is, the first chamber 15 and the liquid storage portion 24 communicate with each other, and the liquid storage portion 24 and the second chamber 16 do not communicate with each other.

Next, the test container 10 is rotated by the centrifuge 110 of the test device 100 and the sample solution 40 is fed from the first chamber 15 to the liquid storage portion 24 of the valve 20 (Step ST16). The reagent 32 is stored in the liquid storage portion 24, and the fed sample solution 40 and the reagent 32 are mixed with each other in the liquid storage portion 24. The reagent 32 is a lyophilized reagent, and is dissolved and mixed with the sample solution 40.

After the sample solution 40 and the reagent 32 are mixed with each other, the valve 20 is switched to the second state by the valve control unit 124 (see Fig. 14) (Step ST17). That is, the second chamber 16 and the liquid storage portion 24 communicate with each other, and the liquid storage portion 24 and the first chamber 15 do not communicate with each other.

Then, the test container 10 is rotated by the centrifuge 110 of the test device 100 and a liquid mixture 42 of the sample solution 40 and the reagent 32 is fed from the liquid storage portion 24 of the valve 20 to the second chamber 16 (Step ST18).

After the liquid mixture 42 is fed to the second chamber 16, the valve 20 is switched to the third state by the valve control unit 124 (see Fig. 14) (Step ST19). Here, at least the second chamber 16 and the liquid storage portion 24 do not communicate with each other. Neither of the first chamber 15 and the second chamber 16 does not communicate with the liquid storage portion 24 in this example, but it is sufficient as long as the second chamber 16 and the liquid storage portion 24 do not communicate with each other. Accordingly, the valve 20 may be switched to the first state.

Here, an LAL reaction is detected on the liquid mixture 42 by a colorimetric method in a state where the second chamber 16 and the liquid storage portion 24 do not communicate with each other (Step ST20). The detection using the colorimetric method is performed by the detection unit 126 disposed above the second chamber 16. Irradiation light L having a specific wavelength is emitted into the second chamber 16 from the light source 126a via the wavelength selective filter 126b. The photodetector 126c detects the absorbance (or transmittance) of the irradiation light L to detect the LAL reaction.

As described above, the same effect can be obtained using the test container 10 as in the case of the nucleic acid test even in the β-glucan test.

The entire content of the present disclosure of Japanese Patent Application No. 2022-057525, filed March 30, 2022, is incorporated in this specification by reference.

All documents, patent applications, and technical standards disclosed in this specification are incorporated in this specification by reference so that the incorporation of each of the documents, the patent applications, and the technical standards by reference is specific and is as detailed as that in a case where the documents, the patent applications, and the technical standards are described individually.

## Claims

1. A test container including an inlet into which a sample solution is to be put, a first chamber in which the sample solution to be fed from the inlet is stored, and a second chamber in which the sample solution to be fed from the first chamber is stored, the test container being attached to a centrifuge and the sample solution being fed from the first chamber to the second chamber in order by a centrifugal force caused by a rotation of the centrifuge, the test container comprising:
an attachable and detachable lid part that covers the inlet;
a liquid reservoir portion that stores the sample solution put in from the inlet in front of the first chamber;
a valve that is disposed between the first chamber and the second chamber, includes a liquid storage portion temporarily storing the sample solution to be fed from the first chamber to the second chamber, and is capable of switching between a first state where the first chamber and the liquid storage portion communicate with each other and the second chamber and the liquid storage portion do not communicate with each other, a second state where the second chamber and the liquid storage portion communicate with each other and the first chamber and the liquid storage portion do not communicate with each other, and a third state where both of the first chamber and the second chamber do not communicate with the liquid storage portion; and
a sealing part that prevents a back flow of the sample solution after the sample solution is fed from the liquid reservoir portion to the first chamber.

2. The test container according to claim 1,
wherein the sealing part is a hydrophobic substance having a specific gravity lower than that of the sample solution.

3. The test container according to claim 2,
wherein the hydrophobic substance is a low-polarity solvent or a thermoplastic substance.

4. The test container according to claim 2 or 3,
wherein the hydrophobic substance is provided in the liquid reservoir portion or the first chamber.

5. The test container according to claim 2 or 3, further comprising:
a hydrophobic substance storage portion that is provided on a side closer to a rotation axis of the centrifuge than the liquid reservoir portion and stores the hydrophobic substance.

6. The test container according to any one of claims 1 to 5,
wherein, in a case where a volume of the liquid reservoir portion is denoted by V 1, a volume of the first chamber is denoted by V2, a volume of the liquid storage portion is denoted by V3, and a volume of the second chamber is denoted by V4, a relationship of "V1 ≥ V2 ≥ V3 ≥ V4" is satisfied.

7. The test container according to any one of claims 1 to 6,
wherein a reagent to react with the sample solution is stored in the first chamber, the liquid storage portion, or the second chamber.

8. The test container according to claim 7,
wherein the reagent contains an amplification reagent for amplifying a specific nucleic acid sequence and a probe for determining a nucleic acid sequence.

9. The test container according to claim 8,
wherein the probe is a fluorescent probe.

10. A nucleic acid test method using the test container according to claim 9 in which the reagent is stored in the liquid storage portion, the test method comprising:
putting the sample solution in from the inlet of the test container in an initial state where the valve is in the second state or the third state, to store the sample solution in the liquid reservoir portion;
hermetically sealing the inlet with the lid part;
feeding the sample solution from the liquid reservoir portion to the first chamber with a centrifugal force;
heating the first chamber to perform heating treatment of the sample solution in a state where a back flow of the sample solution from the first chamber to the liquid reservoir portion is prevented by the sealing part;
switching the valve to the first state;
feeding the sample solution from the first chamber to the liquid storage portion of the valve with a centrifugal force to mix the sample solution and the reagent in the liquid storage portion;
switching the valve to the second state;
feeding a liquid mixture of the sample solution and the reagent from the liquid storage portion to the second chamber with a centrifugal force;
switching the valve to the third state or the first state;
controlling a temperature of the liquid mixture in the second chamber to amplify the specific nucleic acid sequence contained in the liquid mixture;
irradiating the liquid mixture with excitation light to detect fluorescence generated from the fluorescent probe; and
determining whether or not the specific nucleic acid sequence is present or a concentration of the specific nucleic acid sequence.
